(19) **Europäisches Patentamt / European Patent Office / Office européen des brevets**

(11) **EP 2 143 465 B1**

(12) **EUROPEAN PATENT SPECIFICATION**

(45) Date of publication and mention of the grant of the patent:
**03.10.2012 Bulletin 2012/40**

(51) Int Cl.:
***A61N 1/05*** *(2006.01)*

(21) Application number: **09251749.9**

(22) Date of filing: **07.07.2009**

(54) **Electrode cuffs**

Manschettenelektroden

Electrodes à manchette

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.07.2008 US 217930**

(43) Date of publication of application:
**13.01.2010 Bulletin 2010/02**

(73) Proprietor: **Biocontrol Medical Ltd.**
**56100 Yehud (IL)**

(72) Inventors:
• **Ben-David, Tamir**
**Tel Aviv (IL)**
• **Ayal, Shai**
**Shoham 60850 (IL)**
• **Cohen, Ehud**
**Ganei Tikva 55900 (IL)**

(74) Representative: **White, Duncan Rohan**
**Marks & Clerk LLP**
**90 Long Acre**
**London**
**WC2E 9RA (GB)**

(56) References cited:
EP-A- 1 785 160          WO-A-2008/007360
US-A- 5 634 462          US-A- 5 824 027
US-A1- 2008 132 983

• JAMES D SWEENEY ET AL: "An Asymmetric Two Electrode Cuf for Generation of Unidirectionally Propagated Action Potentials" IEEE TRANSACTIONS ON BIOMEDICAL ENGINEERING, IEEE SERVICE CENTER, PISCATAWAY, NJ, US, vol. BME-19, no. 6, 1 June 1986 (1986-06-01), pages 541-549, XP011173957 ISSN: 0018-9294

**Description**

**FIELD OF THE INVENTION**

The present invention relates generally to electrical stimulation of tissue, and specifically to methods and devices for regulating the stimulation of nerves.

**BACKGROUND OF THE INVENTION**

A number of patents and articles describe methods and devices for stimulating nerves to achieve a desired effect. Often these techniques include a design for an electrode or electrode cuff.

US Patent 6,907,295 to Gross et al., which is assigned to the assignee of the present application, describes apparatus for applying current to a nerve. A cathode is adapted to be placed in a vicinity of a cathodic longitudinal site of the nerve and to apply a cathodic current to the nerve. A primary inhibiting anode is adapted to be placed in a vicinity of a primary anodal longitudinal site of the nerve and to apply a primary anodal current to the nerve. A secondary inhibiting anode is adapted to be placed in a vicinity of a secondary anodal longitudinal site of the nerve and to apply a secondary anodal current to the nerve, the secondary anodal longitudinal sice being closer to the primary anodal longitudinal site than to the cathodic longitudinal site.

US Patent Application Publication 2006/0106441 to Ayal et al., which is assigned to the assignee of the present application, describes apparatus for applying current to a nerve, including a housing, adapted to be placed in a vicinity of the nerve, and at least one cathode and at least one anode, fixed to the housing. The apparatus further includes two or more passive electrodes, fixed to the housing, and a conducting element, which electrically couples the passive electrodes to one another.

US Patents 4,608,985 to Crish et al. and 4,649,936 to Ungar et al., describe electrode cuffs for selectively blocking orthodromic action potentials passing along a nerve trunk, in a manner intended to avoid causing nerve damage.

PCT Patent Publication WO 01/10375 to Felsen et al., describes apparatus for modifying the electrical behavior of nervous tissue. Electrical energy is applied with an electrode to a nerve in order to selectively inhibit propagation of an action potential.

US Patent 5,755,750 to Petruska et al., describes techniques for selectively blocking different size fibers of a nerve by applying direct electric current between an anode and a cathode that is larger than the anode.

US Patent 5,824,027 Hoffer et al., describes a nerve cuff having one or more sets of electrodes for selectively recording electrical activity in a nerve or for selectively stimulating regions of the nerve. Each set of electrodes is located in a longitudinally-extending chamber between a pair of longitudinal ridges which project into the bore of the nerve cuff. The ridges are electrically insulating and serve to improve the selectivity of the nerve cuff. The ridges seal against an outer surface of the nerve without penetrating the nerve. In an embodiment, circumferential end sealing ridges extend around the bore at each end of the longitudinal ridges, and are described as enhancing the electrical and/or fluid isolation between different ones of the longitudinally-extending chambers.

US Patent 4,628,942 to Sweeney et al., describes an annular electrode cuff positioned around a nerve trunk for imposing electrical signals on to the nerve trunk for the purpose of generating unidirectionally propagated action potentials. The electrode cuff includes an annular cathode having a circular passage therethrough of a first diameter. An annular anode has a larger circular passage therethrough of a second diameter, which second diameter is about 1.2 to 3.0 times the first diameter. A non-conductive sheath extends around the anode, cathode, and nerve trunk. The anode and cathode are placed asymmetrically to one side of the non-conductive sheath.

As defined by Rattay, in an article entitled, "Analysis of models for extracellular fiber stimulation," IEEE Transactions on Biomedical Engineering, Vol. 36, no. 2, p. 676 (1989), the activation function (AF) of an unmyelinated axon is the second spatial derivative of the electric potential along an axon. In the region where the activation function is positive, the axon depolarizes, and in the region where the activation function is negative, the axon hyperpolarizes. If the activation function is sufficiently positive, then the depolarization will cause the axon to generate an action potential; similarly, if the activation function is sufficiently negative, then local blocking of action potentials transmission occurs. The activation function depends on the current applied, as well as the geometry of the electrodes and of the axon.

For a given electrode geometry, the equation governing the electrical potential is:

$$\nabla(\sigma \nabla U) = 4\pi j,$$

where U is the potential, $\sigma$ is the conductance tensor specifying the conductance of the various materials (electrode housing, axon, intracellular fluid, etc.), and j is a scalar function representing the current source density specifying the locations of current injection. The activation function is found by solving this partial differential equation for U. If an unmyelinated axon is defined to lie in the z direction, then the activation function is:

$$AF = \frac{\partial^2 U}{\partial z^2} .$$

In a simple, illustrative example of a point electrode located a distance d from the axis of an axon in a uniformly-conducting medium with conductance σ, the two equations above are solvable analytically, to yield:

$$AF = \frac{I_{el}}{4\pi\sigma} \bullet \frac{2z^2 - d^2}{(z^2 + d^2)^{2.5}} ,$$

where $I_{el}$ is the electrode current. It is seen that when σ and d are held constant, and for a constant positive $I_{el}$ (to correspond to anodal current), the minimum value of the activation function is negative, and is attained at z = 0, i.e., at the point on the nerve closest to the source of the anodal current. Thus, the most negative point on the activation function corresponds to the place on a nerve where hyperpolarization is maximized, namely at the point on the nerve closest to the anode.

Additionally, this equation predicts positive "lobes" for the activation function on either side of z = 0, these positive lobes peaking in their values at a distance which is dependent on each of the other parameters in the equation. The positive values of the activation function correspond to areas of depolarization, a phenomenon typically associated with cathodic current, not anodal current. However, it has been shown that excess anodal current does indeed cause the generation of action potentials adjacent to the point on a nerve corresponding to z = 0, and this phenomenon is therefore called the "virtual cathode effect." (An analogous, but reverse phenomenon, the "virtual anode effect" exists responsive to excess cathodic stimulation.)

The Rattay article also describes techniques for calculating the activation function for nerves containing myelinated axons. The activation function in this case varies as a function of the diameter of the axon in question. Thus, the activation function calculated for a 1 micron diameter myelinated axon is different from the activation function calculated for a 10 micron diameter axon.

The following patents may be of interest:

US Patent 6,684,105 to Cohen et al.

US Patent 5,423,872 to Cigaina

US Patent 4,573,481 to Bullara

US Patent 6,230,061 to Hartung

US Patent 5,282,468 to Klepinski

US Patent 4,535,785 to van den Honert et al.

US Patent 5,215,086 to Terry et al.

US Patent 6, 341, 236 to Osorio et al.

US Patent 5,487,756 to Kallesoe et al.

US Patent 5,634,462 to Tyler et al.

US Patent 6,456,866 to Tyler et al.

US Patent 4,602,624 to Naples et al.

US Patent 6,600,956 to Maschino et al.

US Patent 5,199,430 to Fang et al.

The following articles may be of interest:

Ungar IJ et al., "Generation of unidirectionally propagating action potentials using a monopolar electrode cuff," Annals of Biomedical Engineering, 14:437-450 (1986)

Sweeney JD et al., "Ar asymmetric two electrode cuff for generation of unidirectionally propagated action potentials," IEEE Transactions on Biomedical Engineering, vol. BME-33(6) (1986)

Sweeney JD et al., "A nerve cuff technique for selective excitation of peripheral nerve trunk regions, " IEEE Transactions on Biomedical Engineering, 37(7) (1990)

Naples GG et al., "A spiral nerve cuff electrode for peripheral nerve stimulation, " by IEEE Transactions on Biomedical Engineering, 35(11) (1988)

van den Honert C et al., "Generation of unidirectionally propagated action potentials in a peripheral nerve by brief stimuli," Science, 206:1311-1312 (1979)

van den Honert C et al., "A technique for collision block of peripheral nerve: Single stimulus analysis," MP-11, IEEE Trans. Biomed. Eng. 28:373-378 (1981)

van den Honert C et al., "A technique for collision block of peripheral nerve: Frequency dependence," MP-12, IEEE Trans. Biomed. Eng. 28:379-382 (1981)

Rijkhoff NJ et al., "Acute animal studies on the use of anodal block to reduce urethral resistance in sacral root stimulation," IEEE Transactions on Rehabilitation Engineering, 2(2):92-99 (1994)

Mushahwar VK et al., "Muscle recruitment through electrical stimulation of the lumbo-sacral spinal cord," IEEE Trans Rehabil Eng, 8(1):22-9 (2000)

Deurloo KE et al., "Transverse tripolar stimulation of peripheral nerve: a modelling study of spatial selectivity," Med Biol Eng Comput, 36(1) :66-74 (1998)

Tarver WB et al., "Clinical experience with a helical bipolar stimulating lead," Pace, Vol. 15, October, Part II (1992)

Hoffer JA et al., "How to use nerve cuffs to stimulate, record or modulate neural activity," in Neural Prostheses for Restoration of Sensory and Motor Function, Chapin JK et al. (Eds.), CRC Press (1st edition, 2000)

Jones JF et al., " Heart rate responses to selective stimulation of cardiac vagal C fibres in anaesthetized cats, rats and rabbits," J Physiol 489(Pt 1):203-14 (1995)

Evans MS et al., "Intraoperative human vagus nerve compound action potentials," Acta Neurol Scand 110:232-238 (2004)

Fitzpatrick et al., "A nerve cuff design for the selective activation and blocking of myelinated nerve fibers," Ann. Conf. of the IEEE Eng. in Medicine and Biology Soc, 13(2), 906 (1991)

Rijkhoff NJ et al., "Orderly recruitment of motoneurons in an acute rabbit model," Ann. Conf. of the IEEE Eng., Medicine and Biology Soc., 20(5):2564 (1998)

Rijkhoff NJ et al., "Selective stimulation of small diameter nerve fibers in a mixed bundle," Proceedings of the Annual Project Meeting Sensations/Neuros and Mid-Term Review Meeting on the TMR-Network Neuros, April 21-23, 1999, pp. 20-21 (1999)

Baratta R et al., "Orderly stimulation of skeletal muscle motor units with tripolar nerve cuff electrode," IEEE Transactions on Biomedical Engineering, 36(8):836-43 (1989)

The following articles describe techniques using cuff electrodes to selectively excite peripheral nerve fibers distant from an electrode without exciting nerve fibers close to the electrode:

Grill WM et al., "Inversion of the current-distance relationship by transient depolarization," IEEE Trans Biomed Eng, 44(1):1-9 (1997)

Goodall EV et al., "Position-selective activation of peripheral nerve fibers with a cuff electrode," IEEE Trans Biomed Eng, 43(8):851-6 (1996)

Veraart C et al., "Selective control of muscle activation with a multipolar nerve cuff electrode," IEEE Trans Biomed Eng, 40(7):640-53 (1993)

Lertmanorat Z et al., "A novel electrode array for diameter-dependent control of axonal excitability: a simulation study," IEEE Transactions on Biomedical Engineering 51(7):1242-1250 (2004)

## SUMMARY OF THE INVENTION

In an exemplary arrangement, an electrode cuff for applying current to a nerve comprises a housing, which is configured to placed at least partially around the nerve, and a plurality of insulating elements arranged at respective longitudinal positions along the housing such that an inner surface of the housing and pairs of the insulating elements define respective cavities (i.e., spaces surrounded by portions of the cuff) at respective longitudinal positions along the housing. The cuff further comprises one or more electrodes, fixed to the housing in fewer than all of the cavities. In other words, at least one of the cavities defined by a pair of the insulating elements does not have an electrode positioned therein. The electrode cuff is typically configured such that, after placement of the cuff, respective contact surfaces of the insulating elements at least partially come in physical contact with the nerve, or substantially in physical contact with the nerve, e.g., are less than about 0.5 mm from the surface of the nerve. As used in the present application, including in the claims, an "electrode" is an electrically conductive element that includes at least one surface that is not electrically insulated.

Providing the one or more empty cavities results in less physical contact between the contact surfaces of the insulating elements and the nerve for a cuff of a given length, than in a cuff of the same length without such an empty cavity. As a result, providing the empty cavities tends to reduce constriction of the nerve by the cuff, which may reduce side-effects of application of the cuff to the nerve. Providing the empty cavity does not have a material impact on the activation function achieved by the electrode cuff.

For some applications, providing a cuff having an increased length along the nerve is desirable, e.g., because such an increased length provides greater space for a distribution of electrodes that enables achievement of a desired activation function that could not be achieved with a shorter cuff. Providing the empty cavity enables the lengthening of the cuff without a concomitant increase in insulating element contact surface area.

There is therefore provided, in accordance with an embodiment of the present invention, apparatus for application to a nerve of a subject, including:

an electrode cuff, which comprises:

a housing, configured to be placed at least partially around the nerve so as to define an inner surface of the housing that faces the nerve; and

a plurality of insulating elements coupled to the inner surface of the housing at respective insulating element longitudinal positions along the housing, such that the inner surface of the housing and pairs of the insulating elements define a plurality of cavities at respective cavity longitudinal positions along the housing;

the apparatus further comprising:

a plurality of electrodes, which comprise one cathode electrode, one anode electrode and at least one further cathode electrode or anode electrode, the electrodes of said plurality of electrodes being fixed to the housing and
a control unit, coupled to the electrodes, and configured to drive at least a portion of the electrodes to apply a current to the nerve; wherein

at least one of the cavities is an empty cavity that does not have an electrode positioned therein, and wherein the apparatus is characterized in that:

the empty cavity is between and directly adjacent along the cuff to two cavities containing two respective anode electrodes, or two cavities containing two respective cathode electrodes.

Typically, the insulating elements are shaped so as to define respective contact surfaces, and the housing and the insulating elements are configured such that the contact surfaces are positioned less than 0.5 mm from a surface cf the nerve when the housing is placed at least partially around the nerve. For some applications, the housing and the insulating elements are configured such that the contact surfaces at least partially come in physical contact with the nerve when the housing is placed at least partially around the nerve. Typically, a length that at least one of the insulating elements protrudes from the housing toward the nerve when the housing is placed at least partially around the nerve is at least 0.5 mm.

For some applications, the electrodes are fixed to the housing in a number of the cavities, wherein a difference between the number of the cavities and a total number of the cavities is an integer between 1 and 3, inclusive, such that between 1 and 3 of the cavities do not have any of the electrodes fixed therein.

In an embodiment, the housing has a length of between 10 mm and 14 mm, an outer radius of between 4 mm and 8 mm, an inner radius of between 3 mm and 6 mm, the insulating elements have an outer radius of between 3 mm and 6 mm, and an inner radius of between 2 mm and 3.5 mm, and the plurality of insulating elements includes exactly seven insulating elements, respective edges of which are positioned within the cuff at the following respective distances from one end of the cuff: 0.0 mm, between 1.3 and 1.7 mm, between 2.7 and 3.3 mm, between 5.1 and 6.3 mm, between 7.1 and 8.7 mm, between 8.5 and 10.3 mm, and between 10.2 and 12.4 mm, and the insulating elements having the following respective widths: between 0.7 and 0.9 mm, between 0.7 and 0.9 mm, between 1.4 and 1.8 mm, between 0.7 and 0.9 mm, between 0.7 and 0.9 mm, between 1.1 and 1.3 mm, and between 0.7 and 0.9 mm.

For some applications, the one or more cavities may include at least four cavities, and the electrodes may be fixed to the housing in at least three of the cavities.

For some applications, the apparatus further includes two or more passive electrodes, and the apparatus further includes a conducting element, which electrically couples the passive electrodes to one another.

In an embodiment, the plurality of insulating elements includes at least seven insulating elements, which are arranged along the housing such that the inner surface of the housing and the pairs of insulating elements define first, second, third, fourth, fifth, and sixth cavities, the first cavity closest to an end of the housing, the second adjacent to the first, the third adjacent to the second, the fourth adjacent to the third, the fifth adjacent to the fourth, and the sixth adjacent to the fifth; the at least one cathode electrode includes at least one first cathode electrode and at least one second cathode electrode; at least a first one of the passive electrodes is fixed to the housing in the first cavity; the at least one anode electrode is fixed to the housing in the second cavity; the at least one first cathode electrode is fixed to the housing in the third cavity; no electrodes are fixed to the housing in the fourth cavity; the at least one second cathode electrode is fixed to the housing in the fifth cavity; and at least a second one of the passive electrodes is fixed to the housing in the sixth cavity.

## BRIEF DESCRIPTION OF THE DRAWINGS

Fig. 1 is schematic, cross-sectional illustration of an electrode cuff for applying current to a nerve, in accordance with respective embodiments of the present invention;

Fig. 2 is a schematic, cross-sectional illustration of another electrode cuff for applying current to a nerve;

Figs. 3 and 4 are graphs modeling calculated activation functions, respectively, when current is applied using electrode cuffs similar to those shown in Figs. 1 and 2, respectively;

Fig. 5 is a schematic, longitudinal cross-sectional view of another electrode cuff for applying current to a nerve; and

Fig 6 is a schematic, cross-sectional illustration of yet another electrode cuff for applying current to a nerve.

## DETAILED DESCRIPTION OF EMBODIMENTS

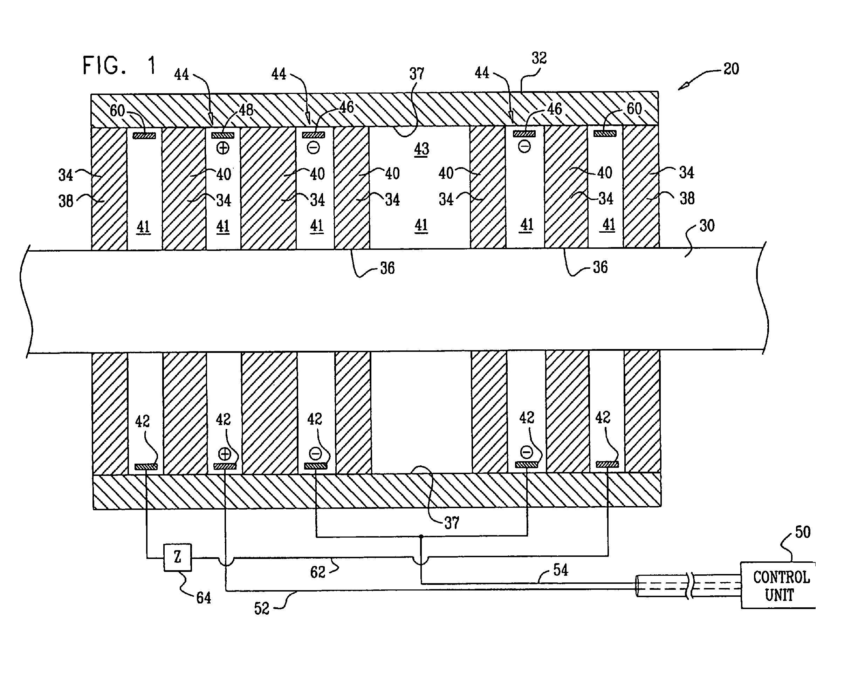

Fig. 1 is a schematic, cross-sectional illustration of an electrode cuff 20 for applying current to a nerve 30, in accordance with an embodiment of the present invention. Electrode cuff 20 comprises a housing 32 which defines an outer surface of the cuff when the cuff is placed at least partially around nerve 30. Housing 32 typically comprises an elastic, electrically-insulating material such as silicone or polyurethane, which may have, for example, a Shore A of between about 35 and about 70, such as about 40.

Electrode cuff 20 further comprises a plurality of insulating elements 34 that are arranged at respective positions along the housing, and are typically fixed to an inner surface 37 of housing 32 that faces nerve 30 when the electrode cuff is placed at least partially around the nerve. Insulating elements 34 typically comprise an elastic, electrically-insulating material such as silicone or silicone copolymer, which, for some applications, is softer than that of housing 32, for example, a Shore A of between about 10 and about 30, such as about 10. Electrode cuff 20 is typically configured such that, after placement of the cuff on the nerve, respective contact surfaces 36 of insulating elements 34 at least partially come in physical contact with the nerve, or substantially in physical contact with the nerve, e.g., are less than about 0.5 mm from the surface of the nerve. For some applications, a length that at least one of insulating elements 34 protrudes from housing 32 toward nerve 30 is at least 0.5 mm, such as at least 1 mm. For some applications, insulating elements 34 and housing 32 are constructed as separate elements that are coupled to one another, while for other applications, the insulating elements and housing are constructed as a single integrated element that is shaped to define the insulating elements and housing.

Insulating elements 34 typically comprise one or more (such as exactly two) end insulating elements 38 arranged at or near respective ends of the cuff, and two or more internal insulating elements 40 arranged at respective positions along the cuff between the end insulating elements. End insulating elements 38 extend along nerve 30 in order to electrically isolate a portion of the nerve within electrode cuff 20 from a portion of the nerve outside the electrode cuff.

Inner surface 37 of housing 32 and pairs of insulating elements 34 define a respective cavities 41 along the housing. (It is noted that some pairs of the insulating elements may not define a cavity, such as if two or more of the insulating elements are arranged in contact with one another.)

Electrode cuff 20 comprises a plurality of electrodes 42, fixed within housing 32 in respective cavities 41 defined by respective pairs insulating elements 34 and inner surface 37 of housing 32. At least one of cavities 41 defined by a pair of the insulating elements does not have an electrode positioned therein. For example, in the embodiment shown in Fig. 1, the insulating elements define six cavities 41, a fourth one 43 of which (counting from the left in the figure) does not have an electrode positioned therein. For some applications, at least two, such as least three, of the cavities do not have electrodes positioned therein. Electrodes 42 are typically fixed to inner surface 37 of housing 32.

For some applications, at least one of the empty cavities has a length along the cuff of at least 0.5 mm, such as at least 0.7 mm, e.g., at least 1.4 mm or at least 2 mm, and/or no more than 5 mm, e.g., no more than 2 mm. For some applications, a length along the cuff of one of the empty cavities is between about 0.5 and about 5 times a length of one of the cavities that has an electrode therein, such as between about 1 and about 2 times the length.

At least one of the empty cavities is directly adjacent along the cuff to two cavities containing two respective anode electrodes, or to two cavities containing two respective cathode electrodes.

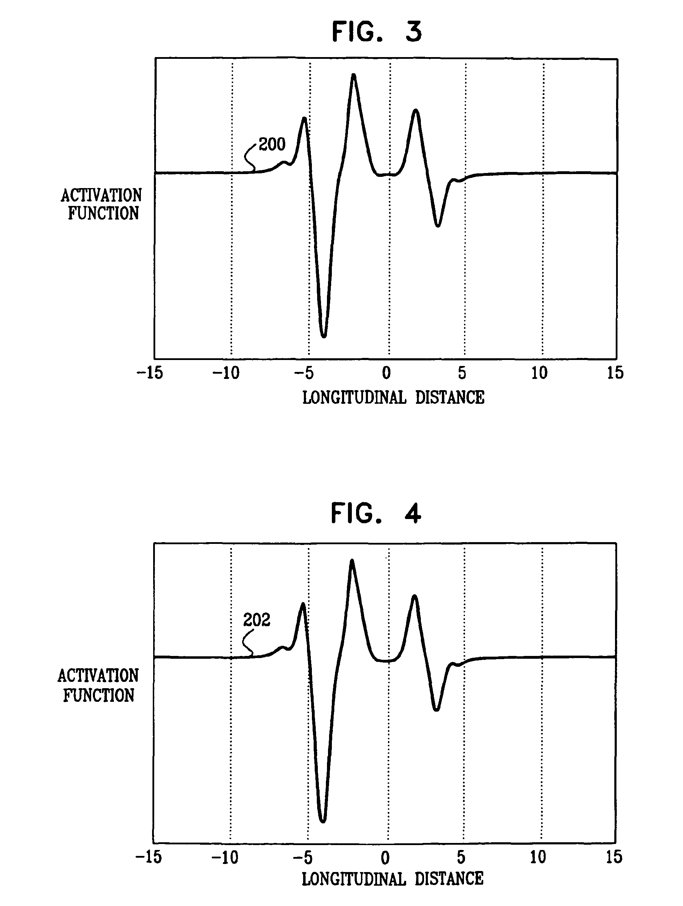

Providing the empty cavity results in less physical contact between contact surfaces 36 of insulating elements 34 and nerve 30 for a cuff of a given length, than in a cuff of the same length without such an empty cavity. As a result, providing the empty cavity tends to reduce constriction of the nerve by the cuff, which may reduce side-effects of application of the cuff to the nerve. Providing the empty cavity does not have a material impact on the activation function achieved by the electrode cuff, as described hereinbelow with reference to Figs. 3 and 4.

Internal insulating elements 40 are arranged so as to electrically separate electrodes 42, and to guide current from one of the electrodes towards the nerve prior to being taken up by another one of the electrodes. Typically (as shown), insulating elements 34 are closer to nerve 30 than are the electrodes, i.e., the electrodes are recessed within the cavities. Alternatively (not shown), insulating elements 34 are generally flush with the faces of the electrodes, such that the inner surfaces of insulating elements and the conductive surfaces of the electrode are equidistant from the nerve.

Electrodes 42 comprise one cathode electrode 46, one anode electrode 48 and at least one further cathode electrode or anode electrode. Active electrodes 44 are coupled to an implantable or external control unit 50 by leads 52 and 54. For some applications, two or more of the active electrodes are shorted to one another inside or outside of the cuff, such as shown for cathode electrodes 46 in Fig. 1.

In an embodiment of the present invention, electrode cuff 20 further comprises two or more passive electrodes 60, fixed within housing 32, and a conducting element 62, typically a wire, which electrically couples the passive electrodes to one another. A "passive electrode," as used in the present application including the claims, is an electrode that is electrically "device-coupled" to neither (a) any circuitry that is electrically device-coupled to any of the cathode electrodes or anode electrodes, nor (b) an energy source. "Device-coupled" means coupled, directly or indirectly, by components of a device, and excludes coupling via tissue of a subject. (It is noted that the passive electrodes may be passive because of a software-controlled setting of the electrode assembly, and that the software may intermittently change the setting such that these electrodes are not passive.) To "passively electrically couple," as used in the present application including the claims, means to couple using at least one passive electrode and no non-passive electrodes. Passive electrodes 60 and conducting element 62 create an additional electrical path for the current, such as an additional path for the current that would otherwise leak outside electrode cuff 20 and travel around the outside of the housing through tissue of the subject. For some applications, conducting element 62 comprises at least one passive element 64, such as a resistor, capacitor, and/or inductor. In this embodiment, end insulating elements 38 help direct any current that leaks from active electrodes 44 through the electrical path created by passive electrodes 60 and conducting element 62. For some applications, active electrodes 44 are positioned within housing 32 longitudinally between the two or more passive electrodes 60 (as shown in Fig. 1). Alternatively, at least one of the passive electrodes is positioned between the at least one cathode electrode and the at least one anode electrode (configuration not shown).

In an embodiment of the present invention, electrode cuff 20 comprises one or more passive electrodes 60 which are not electrically device-coupled to one another. For some applications, the electrode cuff comprises exactly one passive electrode 60. A separate conducting element, typically a wire, is coupled to each passive electrode at a first end of the conducting element. The second end of the conducting element terminates at a relatively-remote location in the body of the subject that is at a distance of at least 1 cm, e.g., at least 2 or 3 cm, from electrode cuff 20. The remote location in the body thus serves as a ground for the passive electrode. For some applications, an electrode is coupled to the remote end of the conducting element, so as to increase electrical contact with tissue at the remote location.

For some applications, housing 32 has a length of between about 10 and about 14 mm, e.g., about 12.1 mm; an outer radius of between about 4 and about 8 mm, e.g., about 5.9 mm; and an inner radius of between about 3 and about 6 mm, e.g., about 4.5 mm. For some applications, insulating elements 34 have an outer radius of between about 3 and about 6 mm, e.g., about 4.5 mm (the outer radius of the insulating elements is typically equal to the inner radius of the housing), and an inner radius of between about 2 and about 3.5 mm. For some applications in which cuff 20 comprises exactly two end insulating elements 38 and exactly five internal insulating elements 40, respective edges of insulating elements 34 are positioned within cuff 32 at the following distances from one end of the cuff: 0.0 mm, between 1.3 and 1.7 mm (e.g., 1.5 mm), between 2.7 and 3.3 mm (e.g., 3.0 mm), between 5.1 and 6.3 mm (e.g., 5.7 mm), between 7.1 and 8.7 mm (e.g., 7.9 mm), between 8.5 and 10.3 mm (e.g., 9.4 mm), and between 10.2 and 12.4 mm (e.g., 11.3 mm), and the insulating elements having the following respective widths: between 0.7 and 0.9 mm (e.g., 0.8 mm), between 0.7 and 0.9 mm (e.g., 0.8 mm), between 1.4 and 1.8 mm (e.g., 1.6 mm), between 0.7 and 0.9 mm (e.g., 0.8 mm), between 0.7 and 0.9 mm (e.g., 0.8 mm), between 1.1 and 1.3 mm (e.g., 1.2 mm), and between 0.7 and 0.9 mm (e.g., 0.8 mm). For some applications, electrodes 42 comprise Pt/Ir. For some applications, as shown in Fig. 1, electrodes 42 are shaped as rings (e.g., reference numeral 60 and leftmost reference numeral 42 in Fig. 1 refer to a single ring electrode). The rings may have an outer radius that equals, or is slightly greater or less than, the inner radius of housing 32.

In an embodiment of the present invention, at least some of the electrodes do not comprise ring electrodes. Instead, each of at least one of non-empty cavities 41 has fixed therein a plurality of electrodes positioned at least partially circumferentially around a central axis of the cuff. In other words, electrodes 42 are first electrodes 42, fixed within housing 32 in respective cavities 41, and cuff 20 comprises at least one second electrode 42, fixed within housing 32 in one of the cavities 41 in which one of the first electrodes 42 is fixed. For some applications, the plurality of electrodes within a single cavity are circumferentially separated from one another by one or more circumferentially arranged insulating elements.

In an embodiment of the present invention, at least one of the one or more of cavities 41 which are empty in the embodiments described hereinabove, instead has fixed therein one or more electrodes that are not electrically device-coupled (as defined hereinabove) to any elements of the device outside of the cavity. These electrodes thus do not serve the normal function of electrodes in an electrode cuff, i.e., conducting current to and/or from tissue.

In an embodiment of the present invention, nerve 30 is a vagus nerve, and electrode cuff 20 is configured to be placed at least partially around the vagus nerve such that anode electrode 48 is more proximal to the brain than are cathode electrodes 46.

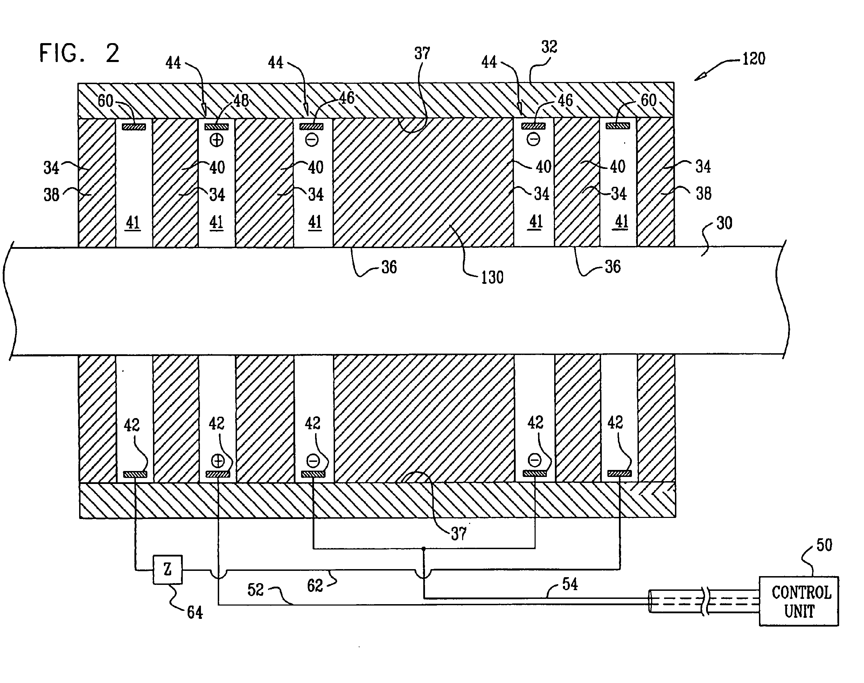

Fig. 2 is a schematic, cross-sectional illustration of an electrode cuff 120 for applying current to nerve 30. Electrode cuff 120 is identical to electrode cuff 20, described hereinabove with reference to Fig. 1, except that cuff 120 lacks cavity 43 of cuff 20, which, as mentioned above, does not have one of electrodes 42 positioned therein. Instead of the two internal insulating elements 40 that define cavity 43 in cuff 20, cuff 120 has a single, elongated insulating element 130, having a length along the housing equal to the sum of the lengths along the cuff of cavity 43 and the two internal insulating elements 40 that define cavity 43 in cuff 20.

Reference is made to Figs. 3 and 4, which are graphs modeling calculated activation functions 200 and 202, respectively, when current is applied using electrode cuffs similar to those shown in Figs. 1 and 2, respectively. These activation functions model myelinated nerve fibers having a diameter of 1 micrometer, over a portion of the length of nerve 30, at a radius of 1.2 mm from the axis of the nerve. For the purposes of modeling these activation functions, (a) two cathode electrodes 46 are placed at longitudinal sites on the nerve labeled $z = 2.25$ mm and $z = -1.65$ mm, respectively, (b) anode electrode 48 is placed at a longitudinal site $z = -4.15$ mm, and (c) two passive electrodes 60 are placed at longitudinal sites $z = 4.15$ mm and $z = -5.65$ mm, respectively. All of the electrodes are placed at a radius of $R = 2.5$ mm from the axis of nerve 30, which has a radius of 1.35 mm. The cavity of activation function 200 (Fig. 3) is at $z = 0.4$ mm.

The inner surfaces of all of the insulating elements (i.e., the surfaces closest to the nerve) are placed at a radius R = 1.5 mm from the axis of nerve 30.

A comparison of activation functions 200 and 202 shows that the two activation functions are nearly identical, which demonstrates that providing empty cavity 43 does not have a material impact on the activation function achieved by the electrode cuff.

For some applications, electrode cuff 20 is configured to selectively stimulate fibers of the nerve having certain diameters, such as by using techniques described in one or more of the patent applications referenced hereinbelow. For example, control unit 50 may drive cathode electrode 46 to apply to nerve 30 a stimulating current, which is capable of inducing action potentials in a first set and a second set of nerve fibers of the nerve, and drive anode electrode 48 to apply to the nerve an inhibiting current, which is capable of inhibiting the induced action potentials traveling in the second set of nerve fibers, the nerve fibers in the second set having generally larger diameters than the nerve fibers in the first set.

For some applications, electrode cuff 20 is configured to apply unidirectional stimulation to the nerve, such as by using techniques described in one or more of the patent applications referenced hereinbelow. For example, control unit 50 may drive anode electrode 48 to apply an inhibiting current capable of inhibiting device-induced action potentials traveling in a non-therapeutic direction in nerve 30. For some applications, electrode cuff 20 comprises primary and secondary anode electrodes, the primary anode electrode located between the secondary anode electrode and the cathode electrode. The secondary anode electrode is typically adapted to apply a current with an amplitude less than about one half an amplitude of a current applied by the primary anode electrode.

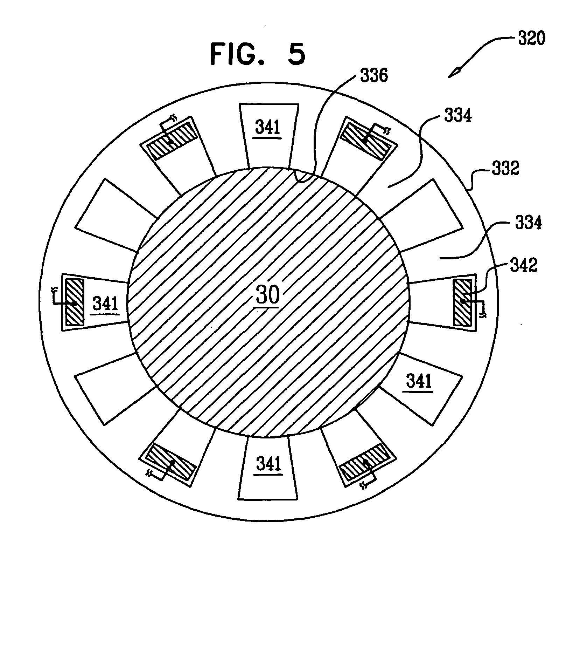

Reference is made to Fig. 5, which is a schematic, cross-sectional view of an electrode cuff 320 for applying current to nerve 30. Electrode cuff 320 comprises a housing 332 which defines an outer surface of the cuff when the cuff is placed at least partially around nerve 30. Housing 332 typically comprises an elastic, electrically-insulating material such as silicone or polyurethane, which may have, for example, a Shore A of between about 35 and about 70, such as about 40. Electrode cuff 20 further comprises a plurality of m insulating elements 334 which are arranged at respective circumferential positions around the housing, and which extend longitudinally along at least a portion of a length of the housing. Insulating elements 334 typically comprise an elastic, electrically-insulating material such as silicone or silicone copolymer, which, for some applications, is softer than that of housing 332, for example, a Shore A of between about 10 and about 30, such as about 10. Electrode cuff 320 is typically configured such that, after placement of the cuff on the nerve, respective contact surfaces 336 of insulating elements 334 come in physical contact with the nerve, cr substantially in physical contact with the nerve, e.g., are less than about 0.5 mm from the surface of the nerve. For some applications, a length that at least one of insulating elements 334 protrudes from housing 332 toward nerve 330 is at least 0.5 mm, such as at least 1 mm. For some applications, insulating elements 334 and housing 332 are constructed as separate elements that are coupled to one another, while for other applications, the insulating elements and housing are constructed as a single integrated element that is shaped to define the insulating elements and housing.

Together, insulating elements 334 define a plurality of n cavities 341 around housing 332, wherein n is less than or equal to m (the number of insulating elements, as mentioned above). Typically, n equals m. Alternatively, n is less than m, such as if two or more of the insulating elements are arranged in contact with one another. It is noted that the cavities 341 of electrode cuff 320 are oriented in a direction that is generally perpendicular to that of cavities 41 of electrode cuff 20 of Fig. 1. Insulating elements 334 of electrode cuff 320 run along the nerve in a direction parallel with a longitudinal axis of the nerve, while insulating elements 34 of electrode cuff 20 surround all or a portion of the nerve.

Electrode cuff 320 comprises a plurality of p electrodes 342, fixed within housing 332 in respective cavities 341 defined by two of insulating elements 334, wherein p is less than n. In other words, at least one of cavities 341 defined by a pair of the insulating elements does not have an electrode positioned therein. For example, in the embodiment shown in Fig. 5, the insulating elements define twelve cavities 341, half of which do not have an electrode positioned therein. For some applications, p equals a fraction of n, such as 2/3, 1/2, 1/3, or 1/4.

For some applications, electrode cuff 320 comprises elements described hereinabove with reference to Fig. 1, such active and/or passive electrodes, and/or a control unit coupled to the cuff with leads.

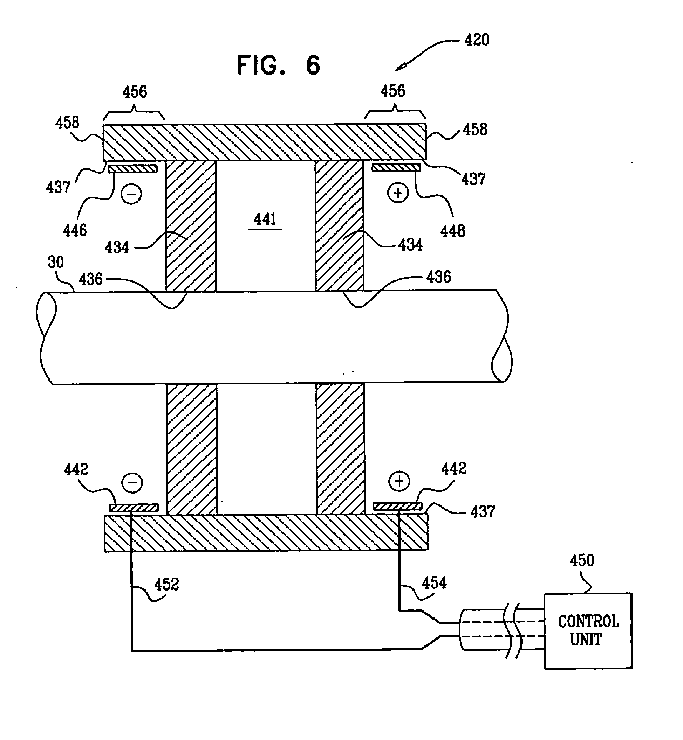

Fig. 6 is a schematic, cross-sectional illustration of an electrode cuff 420 for applying current to nerve 30. Electrode cuff 420 comprises a housing 432 which defines an outer surface of the cuff when the cuff is placed at least partially around nerve 30. Housing 432 typically comprises an elastic, electrically-insulating material such as silicone or polyurethane, which may have, for example, a Shore A of between about 35 and about 70, such as about 40.

Electrode cuff 420 further comprises at least two, e.g., exactly two, insulating elements 434 that are arranged at respective positions along the housing, and are typically fixed to an inner surface 437 of the housing that faces nerve 30 when the cuff is placed at least partially around the nerve. Insulating elements 434 typically comprise an elastic, electrically-insulating material such as silicone or silicone copolymer, which, for some applications, is softer than that of housing 432, for example, a Shore A of between about 10 and about 30, such as about 10. Electrode cuff 420 is typically configured such that, after placement of the cuff on the nerve, respective contact surfaces 436 of insulating elements 434 come in physical contact with the nerve, or substantially in physical contact with the nerve, e.g., are less than about 0.5 mm from the surface of the nerve. For some applications, a length that at least one of insulating elements 434 protrudes from housing 432 toward nerve 30 is at least 0.5 mm, such as at least 1 mm. For some applications, insulating elements 434 and housing 432 are constructed as separate elements that are coupled to one another, while for other applications, the insulating elements and housing are constructed as a single integrated element that is shaped to define the insulating elements and housing. Insulating elements 434 extend along nerve 30 in order to electrically isolate a portion of the nerve within electrode cuff 420 from a portion of the nerve outside the electrode cuff.

Insulating elements 434 are positioned along housing 432 such that end portions 456 of housing 432 extend beyond the insulating elements toward respective longitudinal ends 458 of the housing. In other words, the insulating elements are longitudinally recessed from ends 458 of the housing. In addition, insulating elements 434 are positioned along housing 432 such that inner surface 437 of housing 432 and one or more pairs of the insulating elements define one or more respective cavities 441 along the housing. In the exemplary configuration shown in Fig. 6, the inner surface of the housing and exactly one pair of the insulating elements define exactly one cavity.

Cuff 420 comprises at least two electrodes 442, each of which is fixed to inner surface 437 of housing 432 at at least a portion of one of end portions 456 of housing 432. At least one of cavities 441, e.g., all of cavities 441 and/or exactly one of the cavities, does not have an electrode positioned therein. In other words, the electrodes are fixed to the housing in fewer than all of the cavities, e.g., in none of the cavities. For some applications, at least one of the empty cavities has a length along the cuff of at least 0.5 mm, such as at least 0.7 mm, e.g., at least 1.4 mm or at least 2 mm, and/or no more than 5 mm, e.g., no more than 3 mm or no more than 3 cm.

Providing the empty cavity results in less physical contact between contact surfaces 436 of insulating elements 434 and nerve 30 for a cuff of a given length, than in a cuff of the same length without such an empty cavity. As a result, providing the empty cavity tends to reduce constriction of the nerve by the cuff, which may reduce side-effects of application of the cuff to the nerve. Providing the empty cavity does not have a material impact on the activation function achieved by the electrode cuff.

Electrodes 442 comprise at least one active, i.e., stimulating and/or sensing, electrode, such as at least one cathode electrode 446 and at least one anode electrode 448. The active electrodes are coupled to an implantable or external control unit 450 by leads 452 and 458. For some applications, active electrode configurations and/or stimulation techniques are used which are described in one or more of the patent applications referenced hereinbelow. For some applications, two or more of the active electrodes are shorted to one another inside or outside of the cuff, such as shown for cathode electrodes 46 in Fig. 1. For some applications, cuff 420 comprises one or more passive electrodes, as described hereinabove with reference to Fig. 1.

In an embodiment of the present invention, at least some of electrodes 442 comprise ring electrodes. Alternatively, the electrodes do not comprise ring electrodes. Instead, fixed to at least a portion of each of end portions are a plurality of electrodes positioned at least partially circumferentially around a central axis of the cuff. In other words, electrodes 442 are first electrodes 442, and cuff 420 comprises at least one second electrode 442. For some applications, the plurality of electrodes are circumferentially separated from one another by one or more circumferentially arranged insulating elements.

In an alternative arrangement, at least one of the one or more of cavities 441 which are empty in the arrangement described hereinabove, instead has fixed therein one or more electrodes that are not electrically device-coupled (as defined hereinabove) to any elements of the device outside of the cavity. These electrodes thus do not serve the normal function of electrodes in an electrode cuff, i.e., conducting current to and/or from tissue.

In an alternative arrangement, insulating elements 434 are not positioned so as to define any cavities 441. For example, insulating elements 434 may comprise exactly one insulating element, which may have a length of at least 0.5 mm, such as at least 1 mm.

It is noted that although electrode cuffs 20, 320 and 420 and their elements are generally shown in the figures and described herein in a cylindrical configuration, other geometrical configurations, such as non-rotationally symmetric configurations, are also suitable for applying the principles of the present invention. In particular, housings 32, 332 or 432 of the electrode cuffs (and the electrodes themselves) may form a complete circle around nerve 3C, or they may define an arc between approximately 0 and 90 degrees, between 90 and 180 degrees, between 180 and 350 degrees, or between 350 and 359 degrees around the nerve. For some applications, electrode cuff 20 or 420 comprises electrodes that form rings around the nerve, such that housing 32 surrounds the electrodes.

Techniques described herein can be practiced in combination with techniques described with reference to Figs. 2, 3, and/or 6 of US Patent Application 11/280,884 to Ayal et al., filed November 15, 2005, which published as US Patent Application Publication 2006/0106441, and which is assigned to the assignee of the present application. For example:

• for some applications, a closest distance between cathode electrodes 46 (i.e., the distance between the respective cathode electrodes' edges that are closest to one another) is equal to at least a radius R of nerve 30, e.g., at least 1.5 times the radius of the nerve, as described with reference to Fig. 2 of the '441 publication; and/or

• for some applications, end insulating elements 38 are elongated, as described with reference to Fig. 6 of the '441 publication.

As used in the present patent application, including in the claims, "longitudinal" means along the length of, and does not mean "around" or "circumferential." For example, "longitudinal positions" along the housing means positions along the length of the housing, rather than positions arranged circumferentially around a longitudinal axis of the housing or the nerve. Such longitudinal positions might be measured in mm from one end of the housing.

Techniques and apparatus described in one or more of the following applications can be combined with techniques and apparatus described herein:

- US Provisional Patent Application 60/383,157 to Ayal et al., filed May 23, 2002, entitled, "Inverse recruitment for autonomic nerve systems,"

- International Patent Application PCT / IL02 / 00068 to Cohen et al., filed January 23, 2002, entitled, "Treatment of disorders by unidirectional nerve stimulation," published as WO 03/018113 A1, and US Patent Application 10/488,334, published as US 2004/0243182 A1, in the national stage thereof,

- US Patent Application 09/944,913 to Cohen and Gross, filed August 31, 2001, entitled, "Treatment of disorders by unidirectional nerve stimulation," published as US 2003/0045914 A1, which issued as US Patent 6, 684, 105,

- US Patent Application 09/824,682 to Cohen and Ayal, filed April 4, 2001, entitled "Method and apparatus for selective control of nerve fibers," published as US 2002/0099419 A1,

- US Patent Application 10/205,475 to Gross et al., filed July 24, 2002, entitled, "Selective nerve fiber stimulation for treating heart conditions," published as US 2003/0045909 A1,

- US Patent Application 10/205,474 to Gross et al., filed July 24, 2002, entitled, "Electrode assembly for nerve control," published as US 2003/0050677 A1, which issued as US Patent 6,907,295,

- International Patent Application PCT / IL03 / 00431 to Ayal et al., filed May 23, 2003, entitled, "Selective nerve fiber stimulation for treating heart conditions," published as WO 03/099377 A1,

- International Patent Application PCT / IL03 / 00430 to Ayal et al., filed May 23, 2003, entitled, "Electrode assembly for nerve control," and published as WO 03/099373 A2, US Patent Application 10/529,149, published as US 2006/0116739 A1, in the national stage thereof,

- US Patent Application 10/719,659 to Ben David et al., filed November 20, 2003, entitled, "Selective nerve fiber stimulation for treating heart conditions," published as US 2004/0193231 A1,

- US Patent Application 11/022,011 to Cohen et al., filed December 22, 2004, entitled, "Construction of electrode assembly for nerve control," published as US 2006/0136024 A1,

- US Patent Application 11/234,877 to Ben-David et al., filed September 22, 2005, entitled, "Selective nerve fiber stimulation," published as US 2006/0100668 A1, and

- US Patent Application 11/280,884 to Ayal et al., filed November 15, 2005, entitled, "Techniques for nerve stimulation," which published as US Patent Application Publication 2006/0106441.

## Claims

1. Apparatus for application to a nerve (30) of a subject, comprising:

   an electrode cuff (20), which comprises:

      a housing (32), configured to be placed at least partially around the nerve (30) so as to define an inner surface (37) of the housing (32) that faces the nerve (30) ; and
      a plurality of insulating elements (34) coupled to the inner surface (37) of the housing (32) at respective insulating element longitudinal positions along the housing (32), such that the inner surface (37) of the housing (32) and pairs of the insulating elements (34) define a plurality of respective cavities (41) at respective cavity longitudinal positions along the housing (32);
the apparatus further comprising:

a plurality of electrodes (42), which comprise one cathode electrode (46), one anode electrode (4B) and at least one further cathode electrode (46) or anode electrode (48), the electrodes (42) of said plurality of electrodes being fixed to the housing, and
a control unit (50), coupled to the electrodes (42), and configured to drive at least a portion of the electrodes (42) to apply a current to the nerve (30);
wherein
at least
one of the cavities (41) is an empty cavity (43) that does not have an electrode positioned therein, and wherein the apparatus is **characterized in that**:
the empty cavity (43) is between and directly adjacent along the cuff (20) to two cavities (41) containing two respective anode electrodes (48), or two cavities (41) containing two respective cathode electrodes (46).

2. The apparatus according to claim 1, wherein the insulating elements (34) are shaped so as to define respective contact surfaces (36), and wherein the housing (32) and the insulating elements (34) are configured such that the contact surfaces (36) are suitable for being positioned less than 0.5 mm from a surface of the nerve (30) when the housing (32) is placed at least partially around the nerve (3C).

3. The apparatus according to claim 1, wherein the insulating elements (34) are shaped so as to define respective contact surfaces (36), and wherein the housing (32) and the insulating elements (34) are configured such that the contact surfaces (36) are suitable for at least partially coming in physical contact with the nerve (30) when the housing (32) is placed at least partially around the nerve (30).

4. The apparatus according to claim 1, wherein the electrodes (42) are fixed to the housing (32) in a number of the cavities (41), wherein a difference between the number of the cavities (41) and a total number of the cavities (41, 43) is an integer between 1 and 3, inclusive, such that between 1 and 3 of the cavities (43) do not have any of the electrodes (42) fixed therein.

5. The apparatus according to claim 1,
wherein the housing (32) has a length of between 10 mm and 14 mm, an outer radius of between 4 mm and 8 mm, an inner radius of between 3 mm and 6 mm,
wherein the insulating elements (34) have an outer radius of between 3 mm and 6 mm, and an inner radius of between 2 mm and 3.5 mm, and
wherein the plurality of insulating elements (34) comprises exactly seven insulating elements (34), respective edges of which are positioned within the housing (32) at the following respective distances from one end of the housing (32): 0.0 mm, between 1.3 and 1.7 mm, between 2.7 and 3.3 mm, between 5.1 and 6.3 mm, between 7.1 and 8.7 mm, between 8.5 and 10.3 mm, and between 10.2 and 12.4 mm, and the insulating elements (34) having the following respective widths: between 0.7 and 0.9 mm, between 0.7 and 0.9 mm, between 1.4 and 1.8 mm, between 0.7 and 0.9 mm, between C.7 and 0.9 mm, between 1.1 and 1.3 mm, and between C.7 and 0.9 mm.

6. The apparatus according to claim 1, wherein the electrodes (42) comprise ring electrodes.

7. The apparatus according to claim 1, wherein the one or more cavities (42) include at least four cavities (42), and wherein the electrodes (42) are fixed to the housing (32) in at least three of the cavities (41).

8. The apparatus according to claim 1, wherein the apparatus further comprises two or more passive electrodes (60), and wherein the apparatus further comprises a conducting element (62), which electrically couples the passive electrodes (60) to one another.

9. The apparatus according to claim 8,
wherein the plurality of insulating elements (34) includes at least seven insulating elements (34), which are arranged along the housing (32) such that the inner surface of the housing (32) and the pairs of insulating elements (34) define first, second, third, fourth, fifth, and sixth cavities, the first cavity closest to an end of the housing (32), the second adjacent to the first, the third adjacent to the second, the fourth adjacent to the third, the fifth adjacent to the fourth, and the sixth adjacent to the fifth,

wherein the at least one cathode electrode (46) comprises at least one first cathode electrode (46) and at least one second cathode electrode (46),
wherein at least a first one of the passive electrodes (60) is fixed to the housing (32) in the first cavity,
wherein the at least one anode electrode (48) is fixed to the housing (32) in the second cavity,
wherein the at least one first cathode electrode (46) is fixed to the housing (32) in the third cavity,
wherein no electrodes are fixed to the housing (32) in the fourth cavity (43),
wherein the at least one second cathode electrode (46) is fixed to the housing (32) in the fifth cavity, and
wherein at least a second one of the passive electrodes (60) is fixed to the housing (32) in the sixth cavity.

10. The apparatus according to claim 1, wherein a length that at least one of the insulating elements (34) protrudes from the housing (32) toward the nerve (30) when the housing (32) is placed at least partially around the nerve (30) is at least 0.5 mm.

11. The apparatus according to claim 1, wherein at least two of the electrodes (42) are fixed to the housing (32) in one of the cavities 41).

**Patentansprüche**

1. Vorrichtung zum Anlegen an einen Nerv (30) eines Individuums, umfassend:

    eine Elektrodenmanschette (20), die umfasst:

        ein Gehäuse (32), das konfiguriert ist, um zumindest teilweise um den Nerv (30) platziert zu werden, um eine Innenfläche (37) des Gehäuses (32) zu definieren, die dem Nerv (30) zugewandt ist; und
        eine Mehrzahl von Isolierelementen (34), die mit der Innenfläche (37) des Gehäuses (32) an jeweiligen Isolierelement-Längspositionen entlang des Gehäuses (32) verbunden sind, so dass die Innenfläche (37) des Gehäuses (32) und Paare von Isolierelementen (34) eine Mehrzahl von jeweiligen Hohlräumen (41) an jeweiligen Hohlraum-Längspositionen entlang des Gehäuses (32) definieren;
        wobei die Vorrichtung ferner umfasst:

            eine Mehrzahl von Elektroden (42), die eine Kathodenelektrode (46), eine Anodenelektrode (48) und zumindest eine weitere Kathodenelektrode (46) oder Anodenelektrode (48) umfasst, wobei die Elektroden (42) der Mehrzahl von Elektroden am Gehäuse befestigt sind, und
            eine Steuereinheit (50), die mit den Elektroden (42) verbunden und konfiguriert ist, um zumindest einen Teil der Elektroden (42) anzusteuern, um einen Strom an den Nerv (30) anzulegen;
            wobei zumindest einer der Hohlräume (41) ein leerer Hohlraum (43) ist, in dem keine Elektrode positioniert ist, und wobei die Vorrichtung **dadurch gekennzeichnet ist, dass**:

                der leere Hohlraum (43) zwischen und direkt benachbart entlang der Manschette (20) zu zwei Hohlräumen (41), die zwei jeweilige Anodenelektroden (48) enthalten, oder zwei Hohlräumen (41), die zwei jeweilige Kathodenelektroden (46) enthalten, liegt.

2. Vorrichtung nach Anspruch 1, wobei die Isolierelemente (34) geformt sind, um jeweilige Kontaktflächen (36) zu definieren, und wobei das Gehäuse (32) und die Isolierelemente (34) derart konfiguriert sind, dass die Kontaktflächen (36) geeignet sind, um weniger als 0,5 mm von einer Fläche des Nervs (30) positioniert zu werden, wenn das Gehäuse (32) zumindest teilweise um den Nerv (30) platziert ist.

3. Vorrichtung nach Anspruch 1, wobei die Isolierelemente (34) geformt sind, um jeweilige Kontaktflächen (36) zu definieren, und wobei das Gehäuse (32) und die Isolierelemente (34) derart konfiguriert sind, dass die Kontaktflächen (36) geeignet sind, um zumindest teilweise mit dem Nerv (30) in physikalischen Kontakt zu gelangen, wenn das Gehäuse (32) zumindest teilweise um den Nerv (30) platziert ist.

4. Vorrichtung nach Anspruch 1, wobei die Elektroden (42) am Gehäuse (32) in einer Anzahl der Hohlräume (41) befestigt sind, wobei eine Differenz zwischen der Anzahl der Hohlräume (41) und einer Gesamtanzahl der Hohlräume (41, 43) eine ganze Zahl zwischen 1 und einschließlich 3 ist, so dass in zwischen 1 und 3 der Hohlräume (43) keine der Elektroden (42) befestigt sind.

**5.** Vorrichtung nach Anspruch 1,
wobei das Gehäuse (32) eine Länge zwischen 10 mm und 14 mm, einen Außenradius zwischen 4 mm und 8 mm und einen Innenradius zwischen 3 mm und 6 mm aufweist,
wobei die Isolierelemente (34) einen Außenradius zwischen 3 mm und 6 mm und einen Innenradius zwischen 2 mm und 3,5 mm aufweisen, und
wobei die Mehrzahl von Isolierelementen (34) genau sieben Isolierelemente (34) umfasst, deren jeweilige Enden innerhalb des Gehäuses (32) in den folgenden jeweiligen Abständen von einem Ende des Gehäuses (32) positioniert sind: 0,0 mm, zwischen 1,3 und 1,7 mm, zwischen 2,7 und 3,3 mm, zwischen 5,1 und 6,3 mm, zwischen 7,1 und 8,7 mm, zwischen 8,5 und 10,3 mm und zwischen 10,2 und 12,4 mm, und wobei die Isolierelemente (34) die folgenden jeweiligen Breiten aufweisen: zwischen 0,7 und 0,9 mm, zwischen 0,7 und 0,9 m, zwischen 1,4 und 1,8 mm, zwischen 0,7 und 0,9 mm, zwischen 0,7 und 0,9 mm, zwischen 1,1 und 1,3 mm und zwischen 0,7 und 0,9 mm.

**6.** Vorrichtung nach Anspruch 1, wobei die Elektroden (42) Ringelektroden umfassen.

**7.** Vorrichtung nach Anspruch 1, wobei der eine oder die mehreren Hohlräume (42) zumindest vier Hohlräume (42) umfassen, und wobei die Elektroden (42) am Gehäuse (32) in zumindest drei der Hohlräume (41) befestigt sind.

**8.** Vorrichtung nach Anspruch 1, wobei die Vorrichtung ferner zwei oder mehr passive Elektroden (60) umfasst, und wobei die Vorrichtung ferner ein leitfähiges Element (62) umfasst, das die passiven Elektroden (60) miteinander elektrisch verbindet.

**9.** Vorrichtung nach Anspruch 8,
wobei die Mehrzahl von Isolierelementen (34) zumindest sieben Isolierelemente (34) umfasst, die derart entlang des Gehäuses (32) angeordnet sind, dass die Innenfläche des Gehäuses (32) und die Paare von Isolierelementen (34) einen ersten, zweiten, dritten, vierten, fünften und sechsten Hohlraum definieren, wobei der erste Hohlraum am nächsten zu einem Ende des Gehäuses (32) liegt, wobei der zweite benachbart dem ersten liegt, wobei der dritte benachbart dem zweiten liegt, wobei der vierte benachbart dem dritten liegt, wobei der fünfte benachbart dem vierten liegt und der sechste benachbart dem fünften liegt,
wobei die zumindest eine Kathodenelektrode (46) zumindest eine erste Kathodenelektrode (46) und zumindest eine zweite Kathodenelektrode (46) umfasst,
wobei zumindest eine erste der passiven Elektroden (60) am Gehäuse (32) im ersten Hohlraum befestigt ist,
wobei die zumindest eine Anodenelektrode (48) am Gehäuse (32) im zweiten Hohlraum befestigt ist,
wobei die zumindest eine erste Kathodenelektrode (46) am Gehäuse (32) im dritten Hohlraum befestigt ist,
wobei keine Elektroden am Gehäuse (32) im vierten Hohlraum (43) befestigt sind, wobei die zumindest eine zweite Kathodenelektrode (46) am Gehäuse (32) im fünften Hohlraum befestigt ist, und
wobei zumindest eine zweite der passiven Elektroden (60) am Gehäuse (32) im sechsten Hohlraum befestigt ist.

**10.** Vorrichtung nach Anspruch 1, wobei eine Länge, um die zumindest eines der Isolierelemente (34) vom Gehäuse (32) hin zum Nerv (30) vorspringt, wenn das Gehäuse (32) zumindest teilweise um den Nerv (30) platziert ist, zumindest 0,5 mm beträgt.

**11.** Vorrichtung nach Anspruch 1, wobei zumindest zwei der Elektroden (42) am Gehäuse (32) in einem der Hohlräume (41) platziert sind.

**Revendications**

**1.** Appareil pour une application à un nerf (30) d'un sujet, comprenant :

une manchette à électrodes (20), qui comprend:

un logement (32), configuré pour être placé au moins partiellement autour du nerf (30) de manière à définir une surface intérieure (37) du logement (32) qui est face au nerf (30); et
une pluralité d'éléments d'isolement (34) couplés à la surface intérieure (37) du logement (32) à des positions longitudinales d'éléments d'isolement respectives le long du logement (32), de sorte que la surface intérieure (37) du logement (32) et des paires des éléments d'isolement (34) définissent une pluralité de cavités (41) respectives à des positions longitudinales de cavités respectives le long du logement (32);
l'appareil comprenant en outre:

une pluralité d'électrodes (42), qui comprennent une électrode de cathode (46), une électrode d'anode (48) et au moins une autre électrode de cathode (46) ou électrode d'anode (48), les électrodes (42) de ladite pluralité d'électrodes étant fixées au logement,

et

une unité de commande (50), couplée aux électrodes (42), et configurée pour commander au moins une partie des électrodes (42) pour appliquer un courant au nerf (30);

dans lequel

au moins l'une des cavités (41) est une cavité vide (43) dans laquelle aucune électrode n'est positionnée, et dans lequel l'appareil est **caractérisé en ce que**:

la cavité vide (43) est entre, et directement adjacente le long de la manchette (20), à deux cavités (41) contenant deux électrodes d'anode (48) respectives, ou à deux cavités (41) contenant deux électrodes de cathode (46) respectives.

2. Appareil selon la revendication 1, dans lequel les éléments d'isolement (34) sont formés de manière à définir des surfaces de contact (36) respectives, et dans lequel le logement (32) et les éléments d'isolement (34) sont configurés de sorte que les surfaces de contact (36) soient appropriées pour être positionnées à moins de 0,5 mm d'une surface du nerf (30) lorsque le logement (32) est placé au moins partiellement autour du nerf (30).

3. Appareil selon la revendication 1, dans lequel les éléments d'isolement (34) sont formés de manière à définir des surfaces de contact (36) respectives, et dans lequel le logement (32) et les éléments d'isolement (34) sont configurés de sorte que les surfaces de contact (36) soient appropriées pour au moins partiellement venir en contact physique avec le nerf (30) lorsque le logement (32) est placé au moins partiellement autour du nerf (30).

4. Appareil selon la revendication 1, dans lequel les électrodes (42) sont fixées au logement (32) dans un certain nombre des cavités (41), dans lequel une différence entre le nombre des cavités (41) et un nombre total des cavités (41, 43) est un entier entre 1 et 3, inclus, de sorte que entre 1 et 3 des cavités (43) n'ont aucune électrode (42) fixée dans celles-ci.

5. Appareil selon la revendication 1,
dans lequel le logement (32) a une longueur entre 10 mm et 14 mm, un rayon extérieur entre 4 mm et 8 mm, un rayon intérieur entre 3 mm et 6 mm,
dans lequel les éléments d'isolement (34) ont un rayon extérieur entre 3 mm et 6 mm, et un rayon intérieur entre 2 mm et 3,5 mm, et
dans lequel la pluralité d'éléments d'isolement (34) comprend exactement sept éléments d'isolement (34) dont des bords respectifs sont positionnés dans le logement (32) aux distances respectives suivantes d'une extrémité du logement (32) : 0,0 mm, entre 1,3 et 1,7 mm, entre 2,7 et 3,3 mm, entre 5,1 et 6,3 mm, entre 7,1 et 8,7 mm, entre 8,5 et 10,3 mm, et entre 10,2 et 12,4 mm, et les éléments d'isolement (34) ayant les largeurs respectives suivantes : entre 0,7 et 0,9 mm, entre 0,7 et 0,9 mm, entre 1,4 et 1,8 mm, entre 0,7 et 0,9 mm, entre 0,7 et 0,9 mm, entre 1,1 et 1,3 mm, et entre 0,7 et 0,9 mm.

6. Appareil selon la revendication 1, dans lequel les électrodes (42) comprennent des électrodes annulaires.

7. Appareil selon la revendication 1, dans lequel lesdites une ou plusieurs cavités (42) comprennent au moins quatre cavités (42), et dans lequel les électrodes (42) sont fixées au logement (32) dans au moins trois des cavités (41).

8. Appareil selon la revendication 1, dans lequel l'appareil comprend en outre deux électrodes passives (60) ou plus, et dans lequel l'appareil comprend en outre un élément conducteur (62), qui couple électriquement les électrodes passives (60) les unes aux autres.

9. Appareil selon la revendication 8,
dans lequel la pluralité d'éléments d'isolement (34) comprennent au moins sept éléments d'isolement (34), qui sont agencés le long du logement (32) de sorte que la surface intérieure du logement (32) et les paires d'éléments d'isolement (34) définissent des première, deuxième, troisième, quatrième, cinquième et sixième cavités, la première cavité étant la plus proche d'une extrémité du logement (32), la deuxième étant adjacente à la première, la troisième étant adjacente à la deuxième, le quatrième étant adjacente à la troisième, la cinquième étant adjacente à la quatrième, et la sixième étant adjacente à la cinquième,
dans lequel ladite au moins une électrode de cathode (46) comprend au moins une première électrode de cathode (46) et au moins une deuxième électrode de cathode (46),

dans lequel au moins une première électrode parmi les électrodes passives (60) est fixée au logement (32) dans la première cavité,

dans lequel ladite au moins une électrode d'anode (48) est fixée au logement (32) dans la deuxième cavité,

dans lequel ladite au moins une première électrode de cathode (46) est fixée au logement (32) dans la troisième cavité,

dans lequel aucune électrode n'est fixée au logement (32) dans la quatrième cavité (43),

dans lequel ladite au moins une deuxième électrode de cathode (46) est fixée au logement (32) dans la cinquième cavité, et

dans lequel au moins une deuxième électrode parmi les électrodes passives (60) est fixée au logement (32) dans la sixième cavité.

10. Appareil selon la revendication 1, dans lequel une longueur de laquelle au moins l'un des éléments d'isolement (34) fait saillie du logement (32) vers le nerf (30) lorsque le logement (32) est placé au moins partiellement autour du nerf (30) est d'au moins 0,5 mm.

11. Appareil selon la revendication 1, dans lequel au moins deux des électrodes (42) sont fixées au logement (32) dans l'une des cavités (41).

FIG. 1

FIG. 2

EP 2 143 465 B1

## FIG. 3

ACTIVATION
FUNCTION

LONGITUDINAL DISTANCE

## FIG. 4

ACTIVATION
FUNCTION

LONGITUDINAL DISTANCE

# FIG. 5

## FIG. 6

420

456    456
458    458
437    437
446    448
(−)    (+)
441
434    434
30
436    436

442    442
(−)    (+)
437

452    454    450

CONTROL
UNIT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6907295 B, Gross
- US 20060106441 A, Ayal
- US 4608985 A, Crish
- US 4649936 A, Ungar
- WO 0110375 A, Felsen
- US 5755750 A, Petruska
- US 5824027 A, Hoffer
- US 4628942 A, Sweeney
- US 6684105 B, Cohen
- US 5423872 A, Cigaina
- US 4573481 A, Bullara
- US 6230061 B, Hartung
- US 5282468 A, Klepinski
- US 4535785 A, van den Honert
- US 5215086 A, Terry
- US 6341236 B, Osorio
- US 5487756 A, Kallesoe
- US 5634462 A, Tyler
- US 6456866 B, Tyler
- US 4602624 A, Naples
- US 6600956 B, Maschino
- US 5199430 A, Fang
- US 11280884 B, Ayal
- US 60383157 B, Ayal
- IL 0200068 W, Cohen
- WO 03018113 A1
- US 10488334 B
- US 20040243182 A1
- US 09944913 B, Cohen and Gross
- US 20030045914 A1
- US 09824682 B, Cohen and Ayal
- US 20020099419 A1
- US 10205475 B, Gross
- US 20030045909 A1
- US 10205474 B, Gross
- US 20030050677 A1
- IL 0300431 W, Ayal
- WO 03099377 A1
- IL 0300430 W, Ayal
- WO 03099373 A2
- US 10529149 B
- US 20060116739 A1
- US 10719659 B, Ben David
- US 20040193231 A1
- US 11022011 B, Cohen
- US 20060136024 A1
- US 11234877 B, Ben-David
- US 20060100668 A1

**Non-patent literature cited in the description**

- **RATTAY.** Analysis of models for extracellular fiber stimulation. *IEEE Transactions on Biomedical Engineering,* 1989, vol. 36 (2), 676
- Generation of unidirectionally propagating action potentials using a monopolar electrode cuff. **UNGAR IJ et al.** Annals of Biomedical Engineering. 1986, vol. 14, 437-450
- **SWEENEY JD et al.** Ar asymmetric two electrode cuff for generation of unidirectionally propagated action potentials. *IEEE Transactions on Biomedical Engineering,* 1986, vol. BME-33 (6
- **SWEENEY JD et al.** A nerve cuff technique for selective excitation of peripheral nerve trunk regions. *IEEE Transactions on Biomedical Engineering,* 1990, vol. 37 (7
- **NAPLES GG et al.** A spiral nerve cuff electrode for peripheral nerve stimulation. *IEEE Transactions on Biomedical Engineering,* 1988, vol. 35 (11
- **VAN DEN HONERT C et al.** Generation of unidirectionally propagated action potentials in a peripheral nerve by brief stimuli. *Science,* 1979, vol. 206, 1311-1312
- **VAN DEN HONERT C et al.** A technique for collision block of peripheral nerve: Single stimulus analysis. *IEEE Trans. Biomed. Eng.,* 1981, vol. 28, 373-378
- **VAN DEN HONERT C et al.** A technique for collision block of peripheral nerve: Frequency dependence. *IEEE Trans. Biomed. Eng.,* 1981, vol. 28, 379-382
- **RIJKHOFF NJ et al.** Acute animal studies on the use of anodal block to reduce urethral resistance in sacral root stimulation. *IEEE Transactions on Rehabilitation Engineering,* 1994, vol. 2 (2), 92-99
- **MUSHAHWAR VK et al.** Muscle recruitment through electrical stimulation of the lumbo-sacral spinal cord. *IEEE Trans Rehabil Eng,* 2000, vol. 8 (1), 22-9

- **DEURLOO KE et al.** Transverse tripolar stimulation of peripheral nerve: a modelling study of spatial selectivity. *Med Biol Eng Comput,* 1998, vol. 36 (1), 66-74
- **TARVER WB et al.** Clinical experience with a helical bipolar stimulating lead. *Pace,* October 1992, vol. 15
- How to use nerve cuffs to stimulate, record or modulate neural activity. **HOFFER JA et al.** Neural Prostheses for Restoration of Sensory and Motor Function. CRC Press, 2000
- **JONES JF et al.** Heart rate responses to selective stimulation of cardiac vagal C fibres in anaesthetized cats, rats and rabbits. *J Physiol,* 1996, vol. 489, 203-14
- **EVANS MS et al.** Intraoperative human vagus nerve compound action potentials. *Acta Neurol Scand,* 2004, vol. 110, 232-238
- **FITZPATRICK et al.** A nerve cuff design for the selective activation and blocking of myelinated nerve fibers. *Ann. Conf. of the IEEE Eng. in Medicine and Biology Soc,* 1991, vol. 13 (2), 906
- **RIJKHOFF NJ et al.** Orderly recruitment of motoneurons in an acute rabbit model. *Ann. Conf. of the IEEE Eng., Medicine and Biology Soc.,* 1998, vol. 20 (5), 2564
- **RIJKHOFF NJ et al.** Selective stimulation of small diameter nerve fibers in a mixed bundle. *Proceedings of the Annual Project Meeting Sensations/Neuros and Mid-Term Review Meeting on the TMR-Network Neuros,* 21 April 1999, 20-21
- **BARATTA R et al.** Orderly stimulation of skeletal muscle motor units with tripolar nerve cuff electrode. *IEEE Transactions on Biomedical Engineering,* 1989, vol. 36 (8), 836-43
- **GRILL WM et al.** Inversion of the current-distance relationship by transient depolarization. *IEEE Trans Biomed Eng,* 1997, vol. 44 (1), 1-9
- **GOODALL EV et al.** Position-selective activation of peripheral nerve fibers with a cuff electrode. *IEEE Trans Biomed Eng,* 1996, vol. 43 (8), 851-6
- **VERAART C et al.** Selective control of muscle activation with a multipolar nerve cuff electrode. *IEEE Trans Biomed Eng,* 1993, vol. 40 (7), 640-53
- **LERTMANORAT Z et al.** A novel electrode array for diameter-dependent control of axonal excitability: a simulation study. *IEEE Transactions on Biomedical Engineering,* 2004, vol. 51 (7), 1242-1250